# NEW EUROPEAN PATENT SPECIFICATION

(11) **EP 2 490 639 B2**
(45) Date of publication and mention of the opposition decision: **18.04.2018**
(45) Mention of the grant of the patent: 12.11.2014
(21) Application number: 10824544.0
(22) Date of filing: 09.09.2010
(51) Int. Cl.: A61F 13/514, A61F 13/493, A61F 13/53, B32B 27/02, A61F 13/49

(54) **DISPOSABLE ABSORBENT GARMENTS EMPLOYING ELASTOMERIC FILM LAMINATE BODY PANELS**
SAUGFÄHIGE EINWEGKLEIDUNGSSTÜCKE MIT ELEMENTEN AUS EINEM ELASTOMERFOLIENLAMINAT
VÊTEMENTS ABSORBANTS JETABLES À PANS DE CORPS EN LAMINÉ DE FILM ÉLASTOMÈRE

(30) Priority: 23.10.2009 US 605062
(43) Date of publication of application: 29.08.2012
(73) Proprietor: Kimberly-Clark Worldwide, Inc., Neenah, Wisconsin 54956 (US)
(72) Inventor: THORSON, Russell, E., Appleton Wisconsin 54914 (US); SCHMOKER, Suzanne, M., Oshkosh Wisconsin 54904 (US); RUMAN, Marcille, F., Oshkosh Wisconsin 54901 (US)
(74) Representative: Davies, Christopher Robert
(86) International application number: PCT/IB2010/054076
(87) International publication number: WO 2011/048512

(56) References cited:
- WO-A1-01/13848
- WO-A1-01/13850
- WO-A1-93/17648
- WO-A1-2004/078083
- WO-A1-2004/093765
- WO-A1-2007/099493
- WO-A1-2007/133146
- WO-A1-2011/064995
- WO-A2-97/30671
- US-A1- 2003 100 876
- US-A1- 2004 243 089
- US-A1- 2004 243 089
- US-A1- 2008 071 240
- US-A1- 2009 088 719
- US-A1- 2009 187 157
- US-B1- 6 478 786
- US-B2- 6 702 798

## Description

### Background

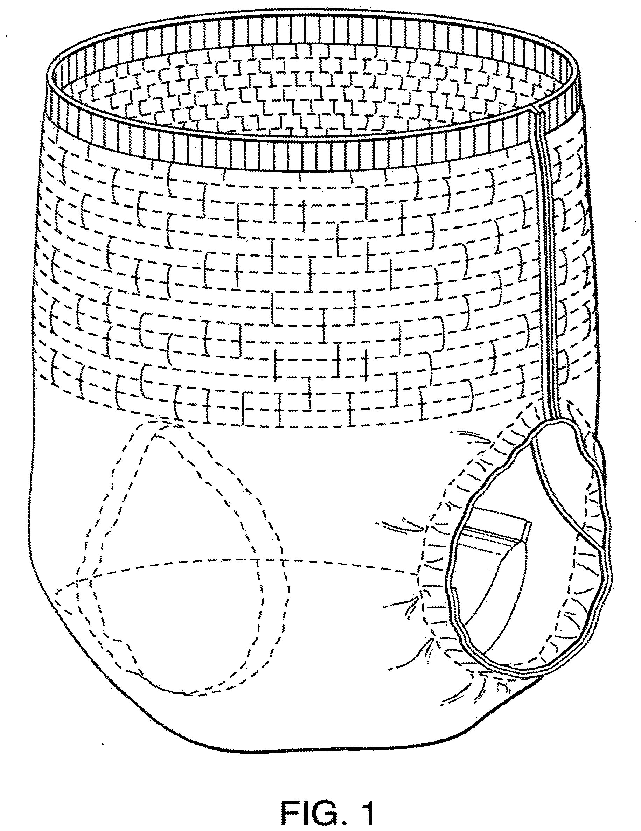

People rely on disposable absorbent products in their everyday lives, including such articles as adult incontinence products, enuresis pants, training pants, and diapers. Many manufacturers seek to better meet the needs of users of such products. For example, there is a need to further improve fit, discretion, and leakage protection for many products. With certain products, such as adult incontinence underwear and enuresis pants, it is important that the garment feel as much as possible like "regular" underwear to promote an improved sense of normalcy to the wearer who suffers from incontinence or enuresis. Many conventional pant-like, pull-on style absorbent garments currently on the market, such as that depicted in Fig. 1, employ a product chassis in which multiple threads of elastic are sandwiched between two nonwoven fabric layers. The strands extend around the body, such that the elastic forces extend primarily around the wearer's waist, as is the case with traditional cloth briefs. This design can provide good fit and leakage performance, but has the potential to be further improved in terms of looking and feeling even more like "regular" cloth underwear.

One class of materials that offers the potential to make absorbent garments more "underwear-like" is that of elastomeric film laminates. Elastomeric film laminates are typically elastomeric films sandwiched between two nonwoven fabric layers. The films provide elastic properties similar to elastic threads, but offer a smoother, more uniform appearance. Some products currently on the market employ such elastomeric laminates, and utilize a "three-piece" pant construction in which front and back body panels are each constructed of an elastomeric film laminate and are connected together via an absorbent insert that extends between them. Such designs, however, can in certain instances introduce a number of drawbacks.

First, many disposable absorbent underwear machines currently in operation in the industry employ a "one-piece" approach to forming the product chassis, in which a "full-length" outer cover layer is formed before the absorbent insert is attached. After the full-length outer cover layer is present in the process, front and back panels are superposed atop the outer cover layer, and elastic threads are sandwiched between the front panel and the outer cover layer, and between the back panel and the outer cover layer. These large and expensive machines are configured primarily to make these "one-piece" pant configurations, and modifying the machines to allow them to make "three-piece" pant configurations would be an expensive, complicated, and time-consuming undertaking. As a result, the "three-piece" pants currently on the market that employ desirable elastomeric films cannot be readily produced on existing "one-piece" pant manufacturing assets. Accordingly, a solution is needed to allow manufacturers to employ existing "one-piece" pant manufacturing assets to produce more underwear-like garments that employ elastomeric film laminates.

Second, certain "three-piece" designs currently on the market that employ elastomeric films suffer from a notable deficiency. The design of certain products results in a region of the pant that is not particularly "underwear-like." Specifically, a region longitudinally between an elasticized waist panel and an elasticized leg edge is devoid of contracting force, and this region tends to bunch and billow during wear. Such billowing and puffing is undesirable for the consumer, as such condition can impact the discretion of the product, as well as negatively affect the fit and aesthetic aspects of the garment.

Therefore, there is a need for improved absorbent garment designs that are compatible with existing manufacturing assets in the industry. Furthermore, there is a need for improved designs of absorbent garments that employ elastomeric film laminates. Finally, there is a need for manufacturing techniques that utilize existing manufacturing capabilities but that provide improved, more underwear-like absorbent garments.

### Summary of the Invention

In response to the aforementioned needs, a new absorbent garment has been invented. The present invention provides a disposable absorbent garment as claimed in claim 1. A disposable absorbent garment defines a longitudinal direction and a transverse direction, and comprises a unitary outer cover layer. The unitary outer cover layer defines a front end edge, a back end edge longitudinally opposite the front end edge, and first and second transversely opposed leg edges positioned longitudinally between the front end edge and the back end edge, each leg edge having a front end point and a back end point. The outer cover defines a front region adjacent the front end edge, a back region adjacent the back end edge, and a crotch region positioned longitudinally between the front region and the back region. The unitary outer cover further defines first and second transversely opposed front side edges, the first front side edge extending in the longitudinal direction from the front end edge to the front end point of the first leg edge, and the second front side edge extending in the longitudinal direction from the front end edge to the front end point of the second leg edge. The unitary outer cover further defines first and second transversely opposed back side edges, the first back side edge extending in the longitudinal direction from the back end edge to the back end point of the first leg edge, and the second back side edge extending in the longitudinal direction from the back end edge to the back end point of the second leg edge. The garment further comprises a first side seam at which the first front side edge is attached to the first back side edge and which defines a first side seam length, and a second side seam at which the second front side edge is attached to the second back side edge and which defines a second side seam length. The garment further comprises an elastomeric front body panel superposed on and attached to the front region of the outer cover layer, the front body panel comprising an elastomeric film laminate that extends transversely from the first front side edge to the second front side edge, that extends longitudinally along the first front side edge a distance at least 50% of the first side seam length, and that extends longitudinally along the second front side edge a distance at least 50% of the second side seam length. The garment further comprises an elastomeric back body panel superposed on and attached to the back region of the outer cover layer, the back body panel comprising an elastomeric film laminate that extends transversely from the first back side edge to the second back side edge, that extends longitudinally along the first back side edge a distance at least 50% of the first side seam length, and that extends longitudinally along the second back side edge a distance at least 50% of the second side seam length. The garment additionally comprises an absorbent assembly superposed over the crotch region of the outer cover layer.

### Definitions

Within the context of this specification, each term or phrase below will include the following meaning or meanings. Additional terms are defined elsewhere in the specification.

"Attached" refers to the joining, adhering, bonding, connecting, or the like, of two elements. Two elements will be considered to be attached together when they are attached directly to one another or indirectly to one another, such as when each is directly attached to intermediate elements.

"Bonded" refers to the joining, adhering, connecting, attaching, or the like, of two elements. Two elements will be considered to be bonded together when they are bonded directly to one another or indirectly to one another, such as when each is directly bonded to intermediate elements.

"Comprising" is inclusive or open-ended and does not exclude additional, unrecited elements or method steps.

"Connected" refers to the joining, adhering, bonding, attaching, or the like, of two elements. Two elements will be considered to be connected together when they are connected directly to one another or indirectly to one another, such as when each is directly connected to intermediate elements.

"Disposable" refers to articles which are designed to be discarded after a limited use rather than being laundered or otherwise restored for reuse.

"Disposed," "disposed on," and variations thereof are intended to mean that one element can be integral with another element, or that one element can be a separate structure bonded to or placed with or placed near another element.

"Elastic," "elasticized" and "elasticity" mean that property of a material or composite by virtue of which it tends to recover its original size and shape after removal of a force causing a deformation.

"Elastomeric" refers to a material or composite which can be elongated by at least percent of its relaxed length and which will recover, upon release of the applied force, at least 20 percent of its elongation. It is generally preferred that the elastomeric material or composite be capable of being elongated by at least 100 percent, more preferably by at least 300 percent, of its relaxed length and recover, upon release of an applied force, at least 50 percent of its elongation.

"Integral" is used to refer to various portions of a single unitary element rather than separate structures bonded to or placed with or placed near one another.

"Layer" when used in the singular can have the dual meaning of a single element or a plurality of elements.

"Longitudinal" and "transverse" have their customary meaning, as indicated by the longitudinal and transverse axes depicted in the Figures. The longitudinal axis lies in the plane of the article and is generally parallel to a vertical plane that bisects a standing wearer into left and right body halves when the article is worn. The transverse axis lies in the plane of the article generally perpendicular to the longitudinal axis. The article as illustrated is longer in the longitudinal direction than in the transverse direction when the article is in a fully stretched and laid-flat condition, prior to the joining of the side seams.

"Member" when used in the singular can have the dual meaning of a single element or a plurality of elements.

These terms may be defined with additional language in the remaining portions of the specification.

### Brief Description of the Drawings

FIG. 1 representatively illustrates a perspective view of a conventional disposable absorbent garment.
FIG. 2 representatively illustrates a front perspective view of a disposable absorbent article incorporating principles of the present invention.
FIG. 3 representatively illustrates a plan view of a disposable absorbent article incorporating principles of the present invention, shown in a longitudinally stretched and laid-flat condition, prior to the joining of the front and back waist regions, and showing the surface of the article that faces toward the wearer when the article is worn.
FIG. 4 is a cross-sectional view of the article of FIG. 3 as viewed along line 4-4.
FIG. 5 is an exploded view of the article of FIGS. 3 and 4.
FIG. 6 is a cross-sectional view of an alternative embodiment of the article of FIG. 3 as viewed along line 4-4.
FIG. 7 is an exploded view of the article of FIG. 6.
FIG. 8 representatively illustrates a front plan view of a disposable absorbent article incorporating principles of the present invention, shown in a fully assembled, longitudinally stretched, and laid-flat condition, and with portions cut away to show underlying features.
FIG. 9 representatively illustrates a front plan view of an alternative embodiment of a disposable absorbent article incorporating principles of the present invention, shown in a fully assembled, longitudinally stretched, and laid-flat condition, and with portions cut away to show underlying features.
FIG. 10 representatively illustrates a front plan view of an alternative embodiment of a disposable absorbent article incorporating principles of the present invention, shown in a fully assembled, longitudinally stretched, and laid-flat condition, and with portions cut away to show underlying features.
FIG. 11 representatively illustrates a plan view of an alternative embodiment of a disposable absorbent article incorporating principles of the present invention, shown in a longitudinally stretched, and laid-flat condition, prior to the joining of the front and back waist regions, and showing the surface of the article that faces toward the wearer when the article is worn.
FIG. 12 is a cross-sectional view of the article of FIG. 11 as viewed along line 12-12.
FIG. 13 is a cross-sectional view of the article of FIG. 11 as viewed along line 13-13.
FIG. 14 is an exploded view of the article of FIG. 12, with certain features omitted.
FIG. 15 representatively illustrates a perspective view of one embodiment of a manufacturing process incorporating principles of the process aspect of the present invention.
FIG. 16 representatively illustrates a perspective view of an alternative embodiment of a manufacturing process incorporating principles of the process aspect of the present invention.
FIG. 17 representatively illustrates a perspective view of another alternative embodiment of a manufacturing outside the scope of the claims.
FIG. 18 representatively illustrates a perspective view of yet another alternative embodiment of a manufacturing process incorporating principles of the process aspect of the present invention.

### Detailed Description of Exemplary Embodiments

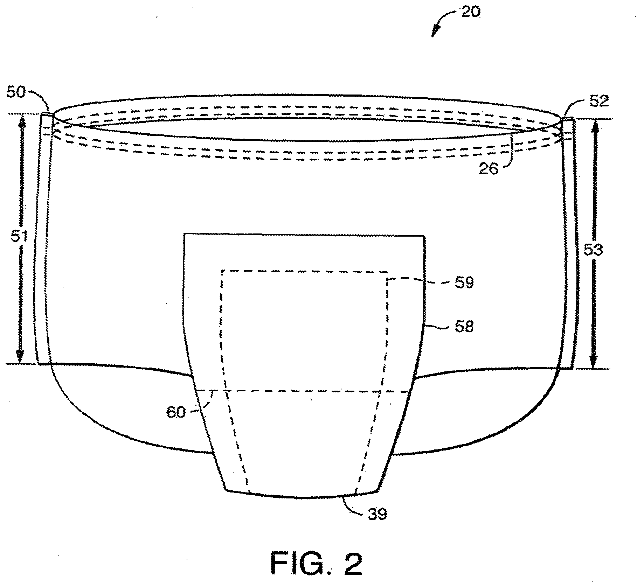

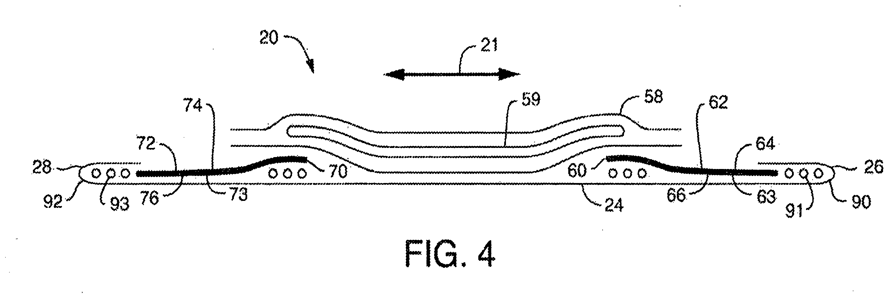

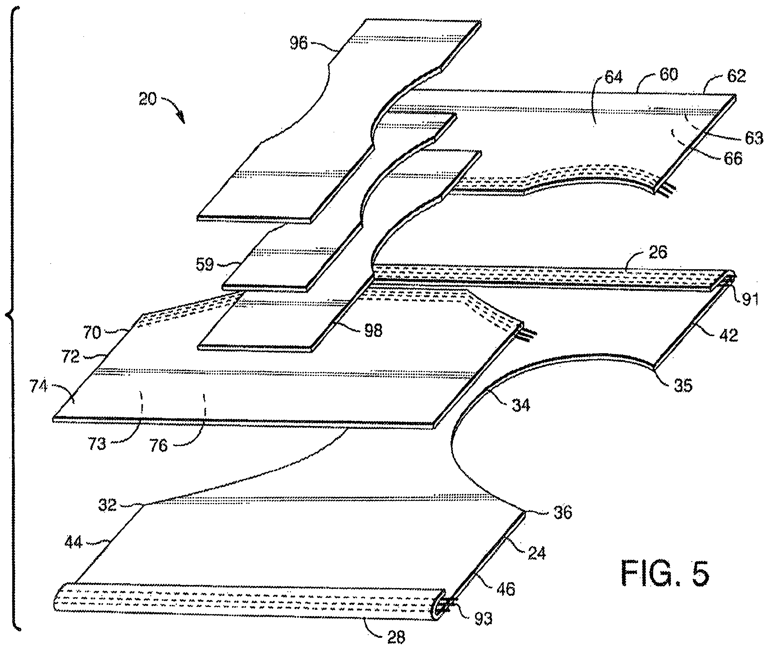

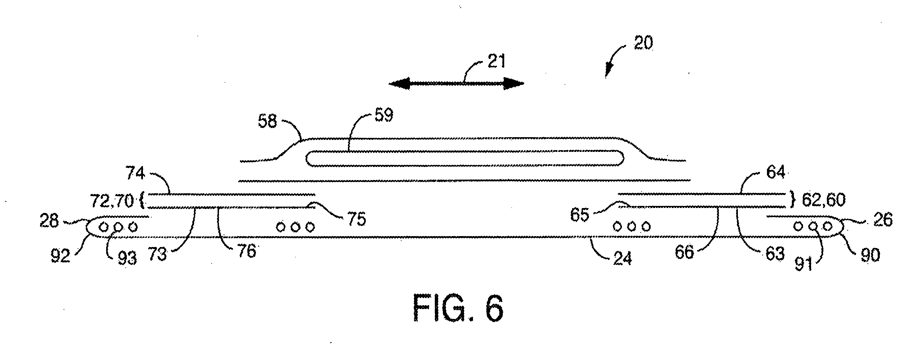

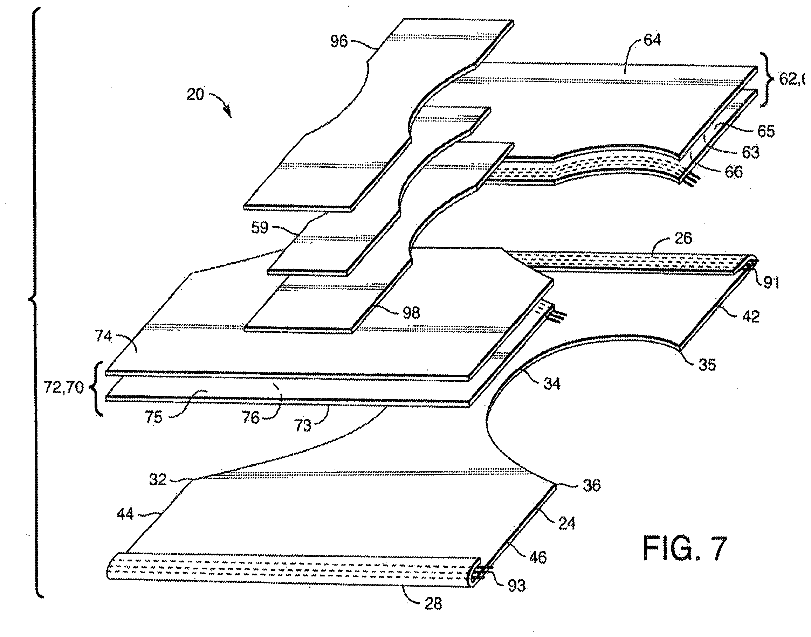

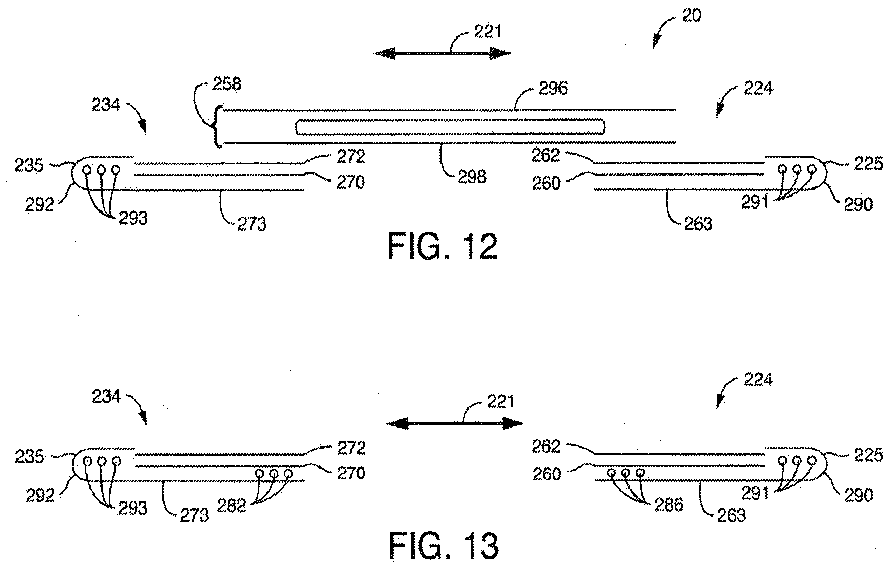

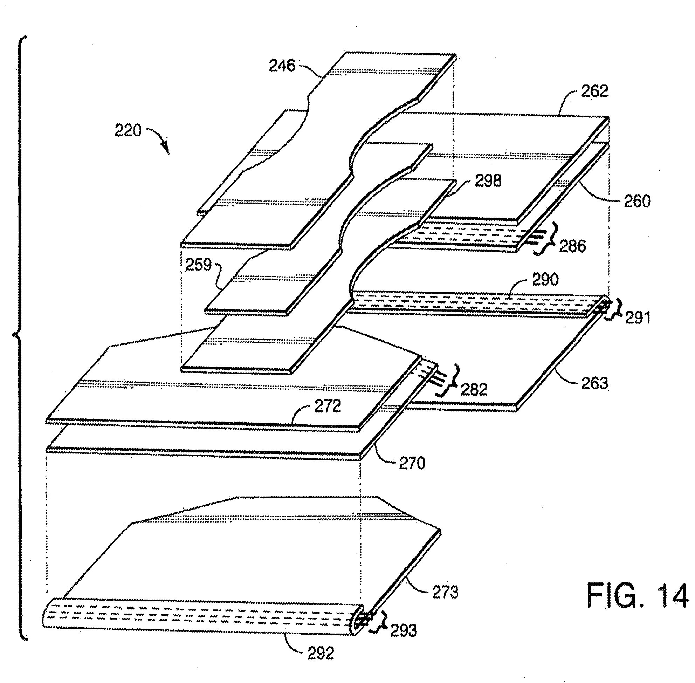

Reference to Figures 2-18 shall be made in describing various aspects and embodiments of the invention. It should be noted that the embodiments depicted in Figures 2-18 are merely representative examples of the garment and process of the invention. Although for illustrative purposes certain features of the present invention shall be described and illustrated with respect to an adult incontinence garment, the various aspects and embodiments of the present invention are also suitable for use with disposable diapers, disposable swim pants, disposable training pants, disposable enuresis garments, and the like.

Referring to Figs. 2-10, a particular embodiment of a disposable absorbent garment 20 of the present invention defines a longitudinal direction 21 and a transverse direction 22, and includes a unitary outer cover layer 24. "Unitary" as used herein means formed from an integral piece of material over substantially its entire area, as opposed to being formed from several pieces that are patched together. The unitary outer cover layer 24 defines a front end edge 26, a back end edge 28 longitudinally opposite the front end edge 26, and first and second transversely opposed leg edges 30 and 34 positioned longitudinally between the front end edge 26 and the back end edge 28. The first leg edge 30 defines a front end point 31 and a back end point 32, and the second leg edge 34 defines a front end point 35 and a back end point 36. The outer cover layer 24 defines a front region 27 adjacent the front end edge 26, a back region 29 adjacent the back end edge 28, and a crotch region 38 positioned longitudinally between the front region 27 and back region 29.

The unitary outer cover layer 24 further defines first and second transversely opposed front side edges 40 and 42. The first front side edge 40 extends in the longitudinal direction 21 from the front end edge 26 to the front end point 31 of the first leg edge 30, and the second front side edge 42 extends in the longitudinal direction 21 from the front end edge 26 to the front end point 35 of the second leg edge 34. The unitary outer cover layer 24 also defines first and second transversely opposed back side edges 44 and 46. The first back side edge 44 extends in the longitudinal direction 21 from the back end edge 28 to the back end point 32 of the first leg edge 30, and the second back side edge 46 extends in the longitudinal direction 21 from the back end edge 28 to the back end point 36 of the second leg edge 34.

The garment includes a first side seam 50 at which the first front side edge 40 is attached to the first back side edge 44 and which defines a first side seam length 51. The garment further includes a second side seam 52 at which the second front side edge 42 is attached to the second back side edge 46 and which defines a second side seam length 53.

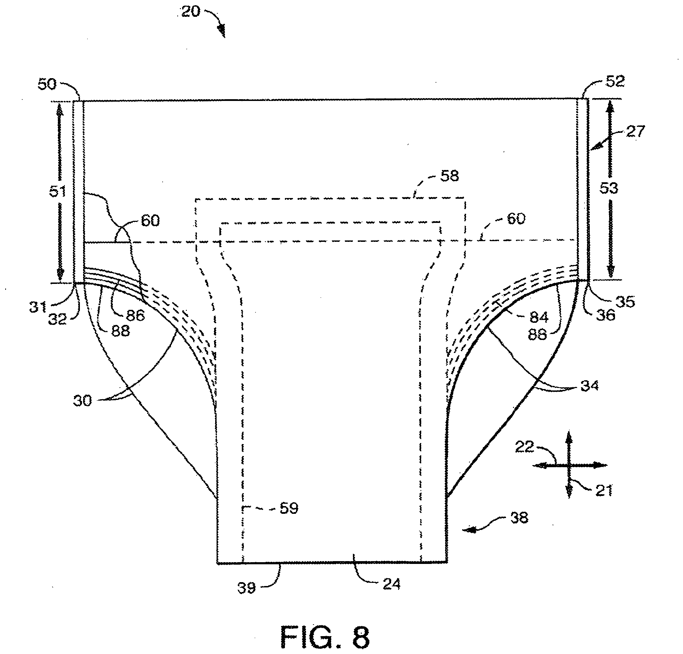

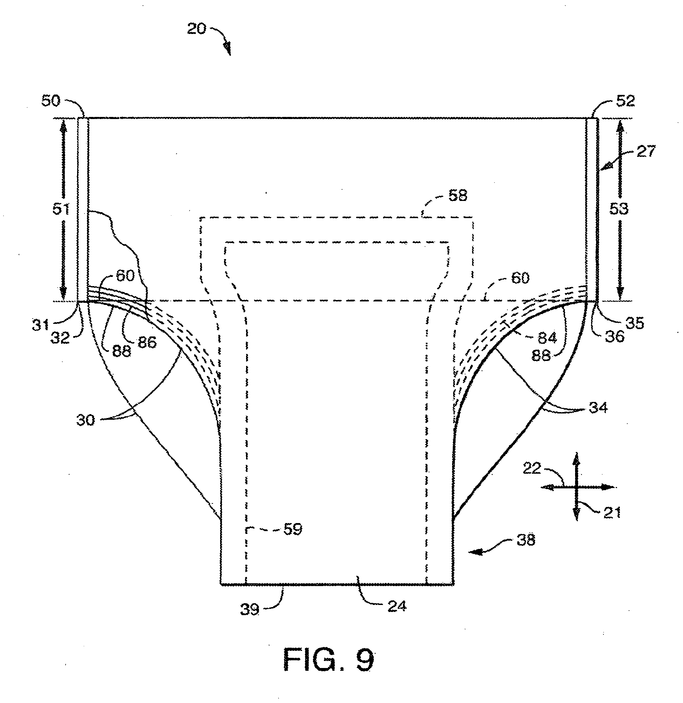

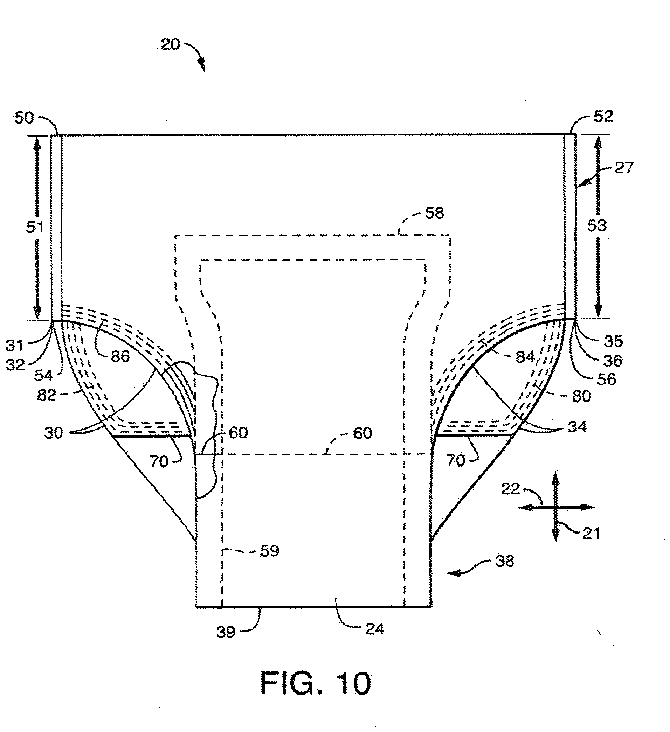

The garment includes an elastomeric front body panel 60 superposed on and attached to the front region 27 of the outer cover 24. The front body panel 60 comprises an elastomeric film laminate 62 that extends transversely from the first front side edge 40 to the second front side edge 42, that extends longitudinally along the first front side edge 40 a distance at least 50% (for example, FIG. 8), more particularly at least 80%, and still more particularly about 100% (for example, FIG. 10) of the first side seam length 51, and that extends longitudinally along the second front side edge 42 a distance at least 50% (for example, FIG. 8), more particularly at least 80%, and still more particularly about 100% (for example, FIG. 9) of the second side seam length 53. The garment also includes an elastomeric back body panel 70 superposed on and attached to the back region 29 of the outer cover 24. As with the front body panel 60 described above, the back body panel 70 comprises an elastomeric film laminate 72 that extends transversely from the first back side edge 44 to the second back side edge 46, that extends longitudinally along the first back side edge 44 a distance at least 50%, more particularly at least 80%, and still more particularly about 100% of the first side seam length 51, and that extends longitudinally along the second back side edge 46 a distance at least 50%, more particularly at least 80%, and still more particularly about 100% of the second side seam length 53. The garment also includes an absorbent assembly 58 superposed over the crotch region 38 of the outer cover layer 24. The absorbent assembly includes an absorbent core 59, and in particular embodiments includes a bodyside liner 96 and a backsheet 98. U.S. Patent Application Publications US 2008/0095978 and US 2009/0197041, both assigned to Kimberly-Clark Worldwide, Inc., provide examples of technology suitable for use in creating the front and back body panel elastomeric film laminates, although other elastomeric film laminates can also be used.

In certain embodiments, as representatively illustrated in Fig. 10, a portion of the front body panel 60 extends longitudinally inward past both the longitudinally innermost point 54 of the first side seam 50 and the longitudinally innermost point 56 of the second side seam 52. Similarly, in certain embodiments and as representatively illustrated in Fig. 10, a portion of the back body panel 70 extends longitudinally inward past both the longitudinally innermost point 54 of the first side seam and the longitudinally innermost point 56 of the second side seam. "Longitudinally inward" as used herein means in a direction longitudinally toward the central transverse axis 23 (if in a laid flat condition prior to the joining of the front and back regions) or in a direction longitudinally toward the crotch fold 39 (if in a fully assembled condition). "Longitudinally outward" as used herein means in a direction longitudinally away from the central transverse axis 23 (if in a laid flat condition prior to the joining of the front and back regions) or in a direction longitudinally away from the crotch fold 39 (if in a fully assembled condition). "Longitudinally innermost" as used herein means closest to the central transverse axis 23 (if in a laid flat condition prior to the joining of the front and back regions) or closest to the crotch fold 39 (if in a fully assembled condition).

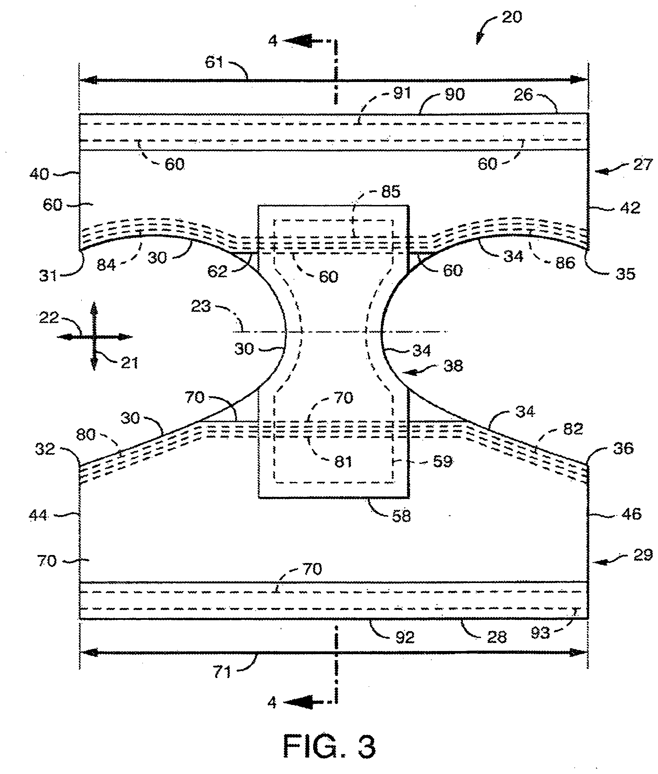

In certain embodiments and as representatively illustrated in Figs. 3-7, the front body panel 60 is longitudinally spaced from and distinct from the back body panel 70. In particular embodiments, as representatively illustrated in Figs. 2-4, 6, and 8-10, at least a portion of the absorbent assembly 58 is superposed over a portion of the front body panel 60. Similarly, in particular embodiments and as representatively illustrated in Figs. 3, 4, and 6, at least a portion of the absorbent assembly 58 is superposed over a portion of the back body panel 70. In such embodiments, it can optionally be the case that a majority of the area of the portion of the front body panel (and/or of the back body panel) superposed by the at least a portion of the absorbent assembly comprises a substantially deadened/ inactivated elastomeric film.

The elastomeric film laminate 62 in the front body panel 60 comprises a front body panel elastomeric film side or layer 63 and a front body panel nonwoven side or layer 64. A bodyside surface 65 of the elastomeric film side or layer 63 is directly adhered to the nonwoven layer 64, and a garment-side surface 66 of the elastomeric film side or layer 63 is directly adhered to the unitary outer cover layer 24, as representatively illustrated in Figs. 3-7. The elastomeric film laminate 72 in the back body panel 70 comprises a back body panel elastomeric film side or layer 73 and a back body panel nonwoven side or layer 74. A bodyside surface 75 of the elastomeric film side layer 73 is directly adhered to the nonwoven layer 74, and a garment-side surface 76 of the elastomeric film side or layer 73 is directly adhered to the unitary outer cover layer 24, as representatively illustrated in Figs. 3-7.

The garment 20 comprises a first back leg elastic member 80 attached to the outer cover layer 24 adjacent at least a portion of the first leg edge 30, and a second back leg elastic member 82 attached to the outer cover layer 24 adjacent at least a portion of the second leg edge 34. In particular embodiments, the garment 20 comprises a first front leg elastic member 84 attached to the outer cover layer 24 adjacent at least a portion of the first leg edge 30, and a second front leg elastic member 86 attached to the outer cover layer 24 adjacent at least a portion of the second leg edge 34. Each leg elastic member 80/82/84/86 can comprise a single strand, ribbon, or strip of elastomeric material, or each can comprise two or more strands, ribbons, or strips, such as, for example, three strands (as depicted in Figs. 3-10).

The first back leg elastic member 80 and the second back leg elastic member 82 are both at least partially sandwiched between the back body panel 70 and the outer cover layer 24. In particular embodiments, as representatively illustrated in Figs. 3 and 10, the first back leg elastic member 80 and the second back leg elastic member 82 are both entirely sandwiched between the back body panel 70 and the outer cover layer 24. Similarly, in certain embodiments, as representatively illustrated in Fig. 9, the first front leg elastic member 84 and the second front leg elastic member 86 are both at least partially sandwiched between the front body panel 60 and the outer cover layer 24. In particular embodiments, as representatively illustrated in Figs. 3 and 10, the first front leg elastic member 84 and the second front leg elastic member 86 are both entirely sandwiched between the front body panel 60 and the outer cover layer 24. In embodiments such as those representatively illustrated in Figs. 8 and 9, in which a leg elastic member (such as front leg elastic members 84 and 86) are not entirely sandwiched between a body panel 60 and the outer cover layer 24, it may be necessary to include a supplemental leg elastic covering layer 88 so that those portions of the leg elastic members 84 and 86 and any concomitant adhesive that are not otherwise fully sandwiched are not exposed. Such a supplemental layer 88 may be formed of suitable nonwoven fabrics as are known in the art.

In certain embodiments, the first back leg elastic member 80 and the second back leg elastic member 82 form part of a single, integral back elastic member 81 that extends from the first back side edge 44 transversely over the absorbent assembly 58 to the second back side edge 46. Similarly, in certain embodiments, the first front leg elastic member 84 and the second front leg elastic member 86 form part of a single, integral front elastic member 85 that extends from the first front side edge 40 transversely over the absorbent assembly 58 to the second front side edge 42.

The front body panel 60 defines a front body panel width 61 that extends transversely from the first front side edge 40 to the second front side edge 42, and the front body panel 60 is longitudinally spaced from the front end edge 26 along the entire front body panel width 61. Similarly, the back body panel 70 defines a back body panel width 71 that extends transversely from the first back side edge 44 to the second back side edge 46, and the back body panel 70 is longitudinally spaced from the back end edge 28 along the entire back body panel width 71. As illustrated in Figs. 3, 4, and 6, the unitary outer cover layer 24 includes a transversely extending front fold 90 that defines the front end edge 26 and a transversely extending back fold 92 that defines the back end edge 28. The garment further includes a front waist elastic member 91 positioned within the front fold 90 and a back waist elastic member 93 positioned within the back fold 92. In illustrative arrangements not in accordance with the present invention, no front waist fold or back waist fold is included; in such arrangements, opposite end edges of the unitary outer cover 24 would define the front end edge 26 of the unitary outer cover 24 and back end edge 28 of the unitary outer cover 24, respectively.

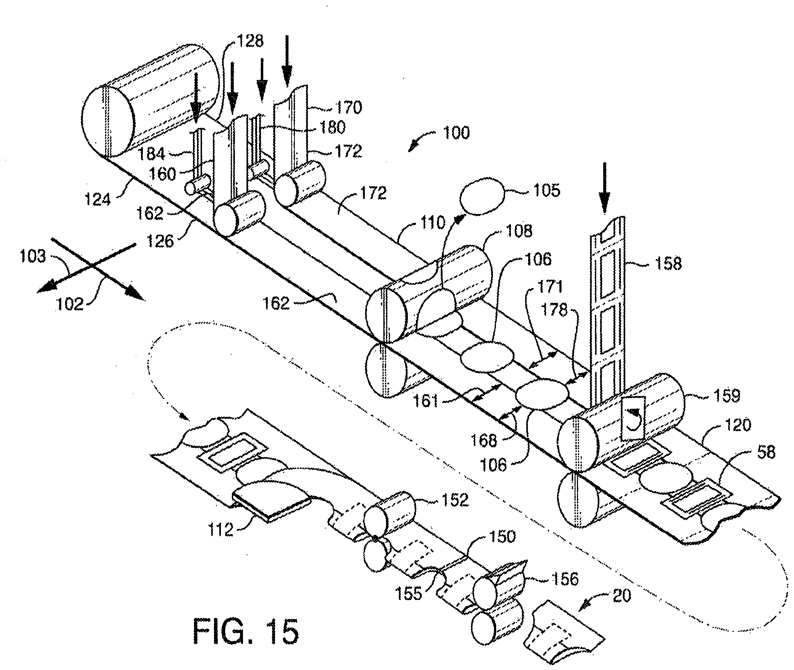

In another aspect, the present invention pertains to a method of manufacturing a plurality of disposable absorbent garments, examples of which are representatively illustrated in Figs. 15-18. In particular embodiments, the method 100 comprises providing an outer cover web 124 traveling in a machine direction 102. The outer cover web has a front waist edge 126 and a back waist edge 128, both of which extend in the machine direction 102.

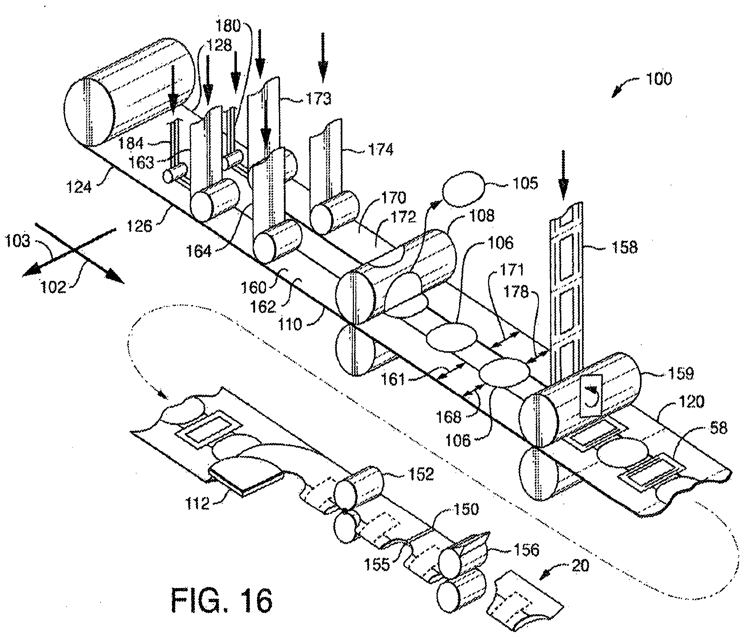

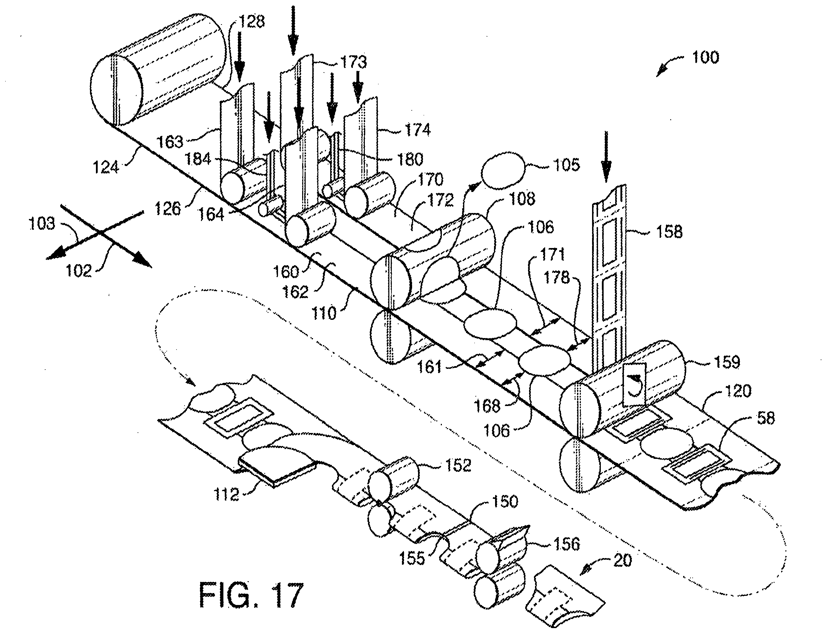

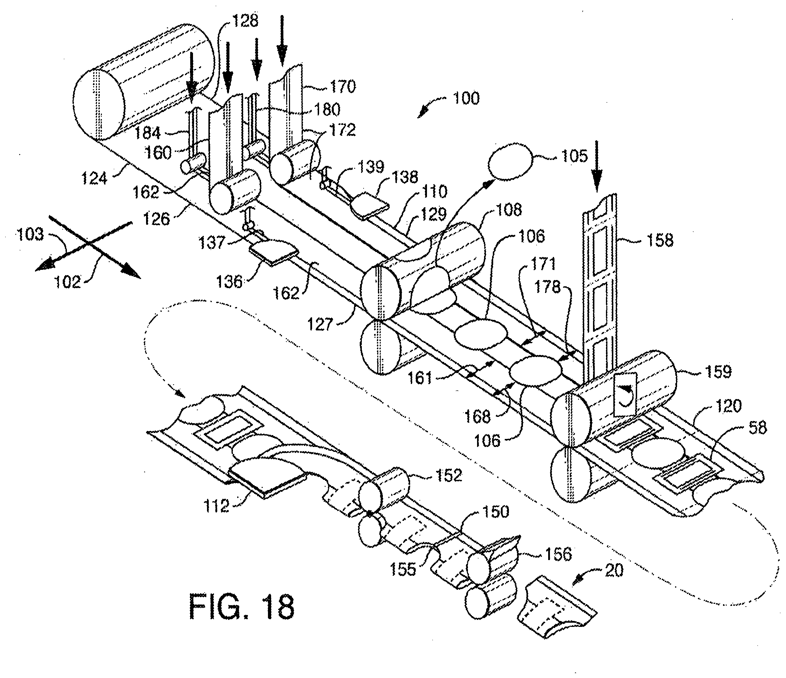

The method in particular embodiments further includes providing an elastomeric front body panel web 160 traveling in the machine direction 102, superposing the elastomeric front body panel web 160 on the outer cover web 124, and attaching the front body panel web 160 to the outer cover web 124. The front body panel web 160 comprises an elastomeric film laminate layer 162. In particular embodiments, the elastomeric film laminate layer 162 includes an elastomeric film web layer 163 and a nonwoven web layer 164 that are separately provided and laminated together during the continuous machine manufacture of the garments. For example, as representatively illustrated in Figs. 16 and 17, the step of providing the elastomeric front body panel web 160 can include providing a roll supply of elastomeric film web layer 163 and providing a separate roll supply of nonwoven web layer 164, superposing the elastomeric film web layer 163 over (or under) the nonwoven web layer 164, and attaching the elastomeric film web layer 163 to the nonwoven web layer 164. In such an embodiment, the step of attaching the elastomeric film web layer 163 to the nonwoven web layer 164 can occur either before or after the composite front body panel web 160 is attached to the outer cover web 124. In alternative embodiments, the elastomeric film laminate 162 includes an elastomeric film web layer 163 and a nonwoven web layer 164 that are laminated together prior to being fed into the continuous machine manufacture of the garments. For example, as representatively illustrated in Figs. 15 and 18, the step of providing the elastomeric front body panel web 160 can include providing a single roll supply of elastomeric film laminate, wherein the laminate on the single roll supply comprises at least two layers, one layer being an elastomeric film web layer 163 and the other layer being a nonwoven layer 164. Note that in Figs. 15 and 18, individual layers of the elastomeric film laminate 162 are not depicted, but instead the laminate is depicted as a single, "pre-made" laminate substrate. In particular embodiments, the front body panel web 160 comprises an elastomeric film layer 162 and a nonwoven layer 164, and the attaching of the front body panel web 160 to the outer cover web 124 comprises adhering the elastomeric film layer 162 of the front body panel web 160 directly to the outer cover web 124, meaning that there are no intervening sheets or films (other than a bonding agent, such as adhesive).

Similarly, the method in particular embodiments further includes providing an elastomeric back body panel web 170 traveling in the machine direction 102, superposing the elastomeric back body panel web 170 on the outer cover web 124, and attaching the back body panel web 170 to the outer cover web 124. The back body panel web 170 comprises an elastomeric film laminate layer 172. In particular embodiments, the elastomeric film laminate 172 includes an elastomeric film web layer 173 and a nonwoven web layer 174 that are separately provided and laminated together during the continuous machine manufacture of the garments. For example, as representatively illustrated in Figs. 16 and 17, the step of providing the elastomeric back body panel web 170 can include providing a roll supply of elastomeric film web layer 173 and providing a separate roll supply of nonwoven web layer 174, superposing the elastomeric film web layer 173 over (or under) the nonwoven web layer 174, and attaching the elastomeric film web layer 173 to the nonwoven web layer 174. In such an embodiment, the step of attaching the elastomeric film web layer 173 to the nonwoven web layer 174 can occur either before or after the back body panel web 170 is attached to the outer cover web 124. In alternative embodiments, the elastomeric film laminate 172 includes an elastomeric film web layer 173 and a nonwoven web layer 174 that are laminated together prior to being fed into the continuous machine manufacture of the garments. For example, as representatively illustrated in Figs. 15 and 18, the step of providing the elastomeric back body panel web 170 can include providing a single roll supply of elastomeric film laminate, wherein the laminate on the single roll supply comprises at least two layers, one layer being an elastomeric film web layer 173 and the other layer being a nonwoven layer 174. Note that in Figs. 15 and 18, individual layers of the elastomeric film laminate 172 are not depicted, but instead the laminate is depicted as a single, "pre-made" laminate substrate. In particular embodiments, the back body panel web 170 comprises an elastomeric film layer 172 and a nonwoven layer 174, and the attaching of the back body panel web 170 to the outer cover web 124 comprises adhering the elastomeric film layer 172 of the back body panel web 170 directly to the outer cover web 124, meaning that there are no intervening sheets or films (other than a bonding agent, such as adhesive).

The outer cover web 124, the front body panel web 160, and the back body panel web 170 collectively define a composite garment web 110. The method in particular embodiments further includes providing a supply 158 of individual absorbent assemblies 58, superposing individual absorbent assemblies 58 over the composite garment web 110, and attaching the individual absorbent assemblies 58 to the composite garment web 110. In certain embodiments, such as those representatively illustrated in Figs. 15-18, the absorbent articles may be manufactured in one orientation, and then cut and rotated 90 degrees (such as at cut-and-rotate station 159) before attachment to the composite garment web 110. The method can in particular embodiments further include removing portions 105 of the outer cover web 124 (such as at cutting station 108) to define a series of spaced apart holes 106, thereby defining in the composite garment web 110 a interconnected series 120 of disposable absorbent garments 20. Such portions 105 can be removed from the outer cover web 124 before the front body panel web 160 and/or back body panel web 170 are attached to the outer cover web 124 (not shown), or can be removed from the outer cover web 124 after the front body panel web 160 and/or back body panel web 170 are attached to the outer cover web 124 (as representatively illustrated in Figs. 15-18). Furthermore, such portions 105 can be removed from the outer cover web 124 before the individual absorbent assemblies 58 are attached to the outer cover web 124 (as representatively illustrated in Figs. 15-18), or can be removed from the outer cover web 124 after the individual absorbent assemblies 58 are attached to the outer cover web 124 (not shown).

The elastomeric front body panel web 160 defines a front body panel web width 161 that extends in a cross-machine direction 103. The front body panel web width 161 extends at least 50%, more particularly at least 70%, still more particularly at least 90%, and yet still more particularly at least 100% of a shortest distance 168 extending from the front waist edge 126 to each hole 106. In one preferred embodiment, such as those representatively illustrated in Figs. 15-18, the front body panel web width 161 exceeds the shortest distance 168 extending from the front waist edge 126 to each hole 106. As the front body panel web width 161 is increased, the resulting garment becomes better elasticized and more underwear-like. In particular embodiments, such as that representatively illustrated in Figs. 15-18, at least a portion of each individual absorbent assembly 58 overlays at least a portion of the elastomeric front body panel web 160.

The elastomeric back body panel web 170 defines a back body panel web width 171 that extends in the cross-machine direction 103. The back body panel web width 171 extends at least 50%, more particularly at least 70%, still more particularly at least 90%, and yet still more particularly at least 100% of a shortest distance 178 extending from the back waist edge 128 to each hole 106. In one preferred embodiment, as representatively illustrated in Figs. 15-18, the back body panel web width 171 exceeds the shortest distance 178 extending from the back waist edge 128 to each hole 106. As the back body panel web width 171 is increased, the resulting garment becomes better elasticized and more underwear-like. In particular embodiments, such as that representatively illustrated in Figs. 15-18, at least a portion of each individual absorbent assembly 58 overlays at least a portion of the elastomeric back body panel web 170.

In particular embodiments, such as that representatively illustrated in Figs. 15-18, after attaching the front body panel web 160 to the outer cover web 124 and after attaching the back body panel web 170 to the outer cover web 124, the front body panel web 160 is spaced in the cross-machine direction 103 apart from the back body panel web 170. In other embodiments, after attaching the front body panel web 160 to the outer cover web 124 and after attaching the back body panel web 170 to the outer cover web 124, the front body panel web 160 can abut or even overlap the back body panel web 170.

The method 100 can further include folding the composite garment web 110, such as at a garment folding station 112, along a transversely centered longitudinal fold line that extends in the machine direction 102, such that the front waist edge 126 is brought into close proximity with the back waist edge 128. In particular embodiments, the method further comprises attaching the front body panel web 160 to the back body panel web 170 to create a series of side seam bonds 150 (such as at seaming station 152) spaced apart in the machine direction 102. The method additionally comprises cutting the composite garment web 110 at a series of cut locations 155 (such as at cutting station 156) spaced apart in the machine direction 102 to create the plurality of disposable absorbent garments.

The method 100 also includes attaching a continuous back leg elastic member 180 to the outer cover web 124. The back leg elastic member 180 extends or travels predominantly in the machine direction 102. As representatively illustrated in Figs. 15, 16 and 18, the method further comprises at least partially overlapping the back leg elastic member 180 with the elastomeric back body panel web 170. The method comprises sandwiching at least a portion of the continuous back leg elastic member 180 between the back body panel web 170 and the outer cover web 124. Similarly, in particular embodiments, the method 100 also includes attaching a continuous front leg elastic member 184 to the outer cover web 124. The front leg elastic member 184 extends or travels predominantly in the machine direction 102. In particular embodiments, as representatively illustrated in Figs. 15-18, the method further comprises at least partially overlapping the front leg elastic member 184 with the elastomeric front body panel web 160. For example, the method may comprise sandwiching at least a portion of the continuous front leg elastic member 184 between the front body panel web 160 and the outer cover web 124. In certain embodiments, the method 100 further comprises cutting the continuous back leg elastic member 180 and/or the continuous front leg elastic member 184 at a series of elastic cut points spaced apart in the machine direction, each elastic cut point being generally aligned in the machine direction 102 with a respective individual absorbent assembly 58 (not shown). Suitable methods for such cutting step are disclosed in U.S. Patent 5,660,657 issued May 5, 1998 to Rajala et al. and assigned to Kimberly-Clark Worldwide, Inc..

In particular embodiments, such as that representatively illustrated in Fig. 18, the method further includes folding the front waist edge 126 of the outer cover web 124, such as at a front waistband folding station 136, to create a front waist edge fold 127 and to encase a front waist elastic member 137. In such an embodiment, the front waist edge fold 127 defines the front waist edge 126 of the outer cover web 124. The method may instead or additionally include folding the back waist edge 128 of the outer cover web 124, such as at back waistband folding station 138, to create a back waist edge fold 129 and to encase a back waist elastic member 139. In such an embodiment, the back waist edge fold 129 defines the back waist edge 128 of the outer cover web 124. Note that in such an embodiment, the front body panel web width 161 and the back body panel width 171 are measured after the creation of the front and back waist edge folds 127/129, as illustrated in Fig. 18.

In particular embodiments, various components, such as the outer cover web 124, one or more layers of the front body panel web 160, and/or one or more layers of the back body panel web 170, can be printed or pigmented to include graphics, text, color, or other images. Such printing can occur during assembly of the garment in conjunction with the presently disclosed method, or can occur prior to such assembly in an off-line, "pre-preprinting" or pigmenting step.

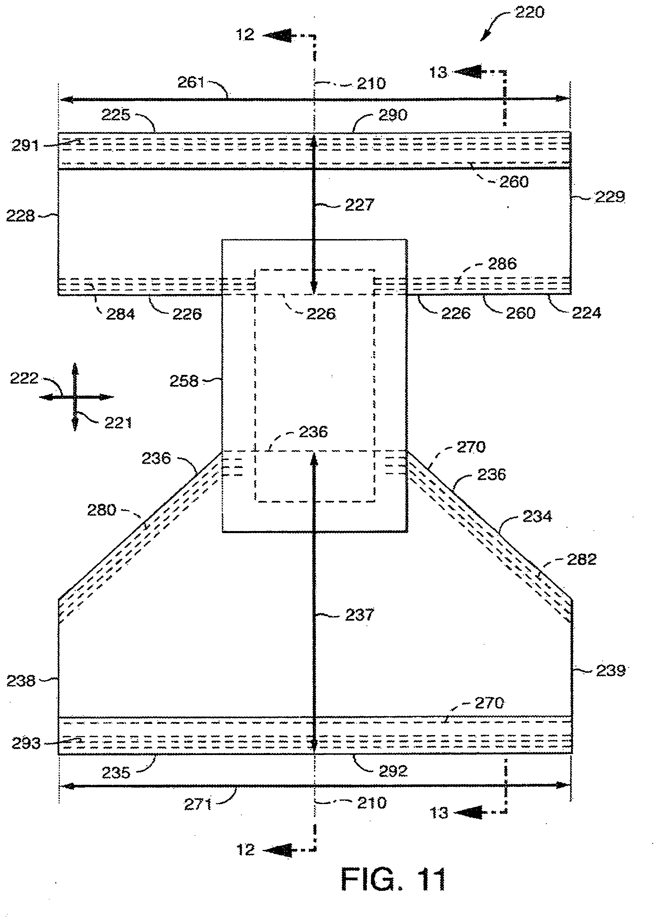

Referring to Figs. 11-14, an alternative embodiment of the garment aspect of the present invention shall now be described. In this embodiment, a disposable absorbent garment 220 defines a longitudinal direction 221 and a transverse direction 222. The garment 220 comprises a front panel 224 which defines a front panel end edge 225, a front panel crotch edge 226 longitudinally opposite the front panel end edge 225, a front panel length 227 extending longitudinally from the front panel end edge 225 to the front panel crotch edge 226 and measured at the longitudinal centerline 210 of the garment 220, and first and second transversely opposed front panel side edges 228 and 229. The first front panel side edge 228 extends in the longitudinal direction 221 from the front panel end edge 225 to the front panel crotch edge 226, and the second front panel side edge 229 extends in the longitudinal direction 221 from the front panel end edge 225 to the front panel crotch edge 226. The front panel 224 comprises a front panel elastomeric film 260 which extends transversely from the first front panel side edge 228 to the second front panel side edge 229. The front panel 224 can further comprise a front panel inner nonwoven layer 262 and a front panel outer nonwoven layer 263. The front panel elastomeric film 260 extends longitudinally at least 70%, more particularly at least 80%, and still more particularly at least 95% of the front panel length 227.

The garment 220 further comprises a back panel 234 which defines a back panel end edge 235, a back panel crotch edge 236 longitudinally opposite the back panel end edge 235, a back panel length 237 extending longitudinally from the back panel end edge 235 to the back panel crotch edge 236 and measured at the longitudinal centerline 210 of the garment 220, and first and second transversely opposed back panel side edges 238 and 239. The first back panel side edge 238 extends in the longitudinal direction 221 from the back panel end edge 235 to the back panel crotch edge 236, and the second back panel side edge 239 extends in the longitudinal direction 221 from the back panel end edge 235 to the back panel crotch edge 236. The back panel 234 comprises a back panel elastomeric film 270 which extends transversely from the first back panel side edge 238 to the second back panel side edge 239. The back panel 234 can further comprise a back panel inner nonwoven layer 272 and a back panel outer nonwoven layer 273. The back panel elastomeric film 270 extends longitudinally at least 70%, more particularly at least 80%, and still more particularly at least 95% of the back panel length 237.

The back panel 234 includes first and second back leg elastic members 280 and 282, each of which extends along at least a portion of the back panel crotch edge 236. Each back leg elastic member 280 and 282 at least partially overlaps the back panel elastomeric film 270. In a particular embodiment, both the first back leg elastic member 280 and the second back leg elastic member 282 are both entirely overlapped by the back panel elastomeric film 270. For example, both the first back leg elastic member 280 and the second back leg elastic member 282 may each be entirely sandwiched between the back panel elastomeric film 270 and a nonwoven facing affixed to the back panel elastomeric film 270.

Similarly, the front panel 224 may include first and second front leg elastic members 284 and 286, each of which extends along at least a portion of the front panel crotch edge 226. In this particular embodiment, each front leg elastic member 284 and 286 at least partially overlaps the front panel elastomeric film 260. In a particular embodiment, both the first front leg elastic member 284 and the second front leg elastic member 286 are both entirely overlapped by the front panel elastomeric film 260. For example, both the first front leg elastic member 284 and the second front leg elastic member 286 may each be entirely sandwiched between the front panel elastomeric film 260 and a nonwoven facing affixed to the front panel elastomeric film 260. Each leg elastic member 280/282/284/286 can comprise a single strand, ribbon, or strip of elastomeric material, or each can comprise two or more strands, ribbons, or strips, such as, for example, three strands (as depicted in Figs. 11-14).

The garment 220 further comprises an absorbent assembly 258 that extends between and interconnects the front panel 224 and the back panel 234. The absorbent assembly can include an absorbent core 259, a liner 296, and a backsheet 298. In particular embodiments, at least a portion of the absorbent assembly 258 is superposed over a portion of the front panel 224. In additional or in the alternative, at least a portion of the absorbent assembly 258 may be superposed over a portion of the back panel 234. In particular embodiments, a majority of the area of the portion of the front panel superposed by the at least a portion of the absorbent assembly comprises a deadened elastomeric film. Suitable configurations for such deadened/deactivated/inactive elastomeric regions are disclosed in U.S. patent application serial no. 12/346060, entitled "Disposable Absorbent Garments Employing Elastomeric Film Laminates With Deactivated Regions" and filed December 30, 2008 in the name of Stabelfeldt et al. and assigned to Kimberly-Clark Worldwide, Inc.. In particular embodiments, the first back leg elastic 280 and the second back leg elastic 282 may form part of a single, integral back elastic that extends transversely over the absorbent assembly 258. In addition or instead, the first front leg elastic 284 and the second front leg elastic 286 may form part of a single, integral front elastic that extends transversely over the absorbent assembly 258. In other embodiments, such as those depicted in Figs. 11-14, the first back leg elastic 280 and the second back leg elastic 282 are disconnected and transversely spaced from each other, such that no back leg elastic component extends transversely over the absorbent assembly 258. In addition or instead, such as is depicted in Figs. 11-14, the first front leg elastic 284 and the second front leg elastic 286 are disconnected and transversely spaced from each other, such that no front leg elastic component extends transversely over the absorbent assembly 258.

In particular embodiments, the front panel elastomeric film 260 defines a front panel elastomeric film width 261, measured at the front panel end edge 225, that extends transversely from the first front panel side edge 228 to the second front panel side edge 229. The front panel elastomeric film 260 can be longitudinally spaced from the front panel end edge 225 along the entire front panel elastomeric film width 261. In such embodiments, the front panel can but need not include a transversely extending front fold 290 that defines the front panel end edge 225, and can include a front waist elastic member 291 positioned within the front fold 290.

Additionally or alternatively, the back panel elastomeric film 270 defines a back panel elastomeric film width 271, measured at the back panel end edge 235, that extends transversely from the first back panel side edge 238 to the second back panel side edge 239. The back panel elastomeric film 270 can be longitudinally spaced from the back panel end edge 235 along the entire back panel elastomeric film width 271. In such embodiments, the back panel can but need not include a transversely extending back fold 292 that defines the back panel end edge 235, and can include a back waist elastic member 293 positioned within the back fold 292. The garment 220 can further include a first side seam at which the first front panel side edge 228 is attached to the first back panel side edge 238, as well as a second side seam at which the second front panel side edge 229 is attached to the second back panel side edge 239.

It will be appreciated that details of the foregoing embodiments, given for purposes of illustration, are not to be construed as limiting the scope of this invention. Although only a few exemplary embodiments of this invention have been described in detail, those skilled in the art will readily appreciate that many modifications are possible in the exemplary embodiments without materially departing from the novel teachings and advantages of this invention. Accordingly, all such modifications are intended to be included within the scope of this invention, which is defined in the following claims and all equivalents thereto. Further, it is recognized that many embodiments may be conceived that do not achieve all of the advantages of some embodiments, particularly of the preferred embodiments, yet the absence of a particular advantage shall not be construed to necessarily mean that such an embodiment is outside the scope of the present invention.

## Claims

1. A disposable absorbent garment (20) defining a longitudinal direction (21) and a transverse direction (22), the garment comprising:
a unitary outer cover layer (24), the unitary outer cover layer (24) defining:
a front end edge (26), a back end edge (28) longitudinally opposite the front end edge (26), and first and second transversely opposed leg edges (30,34) positioned longitudinally between the front end edge (26) and the back end edge (28), each leg edge (30,34) having a front end point (31,35) and a back end point (32,36), the outer cover (24) defining a front region (27) adjacent the front end edge (26), a back region (29) adjacent the back end edge (28), and a crotch region (38) positioned longitudinally between the front region (27) and the back region (29);
first and second transversely opposed front side edges (40,42), the first front side edge (40) extending in the longitudinal direction (21) from the front end edge (26) to the front end point (31) of the first leg edge (30), and the second front side edge (42) extending in the longitudinal direction (21) from the front end edge (26) to the front end point (35) of the second leg edge (34); and
first and second transversely opposed back side edges (44,46), the first back side edge (44) extending in the longitudinal direction (21) from the back end edge (28) to the back end point (32) of the first leg edge (30), and the second back side edge (46) extending in the longitudinal direction (21) from the back end edge (28) to the back end point (36) of the second leg edge (34);
a first side seam (50) at which the first front side edge (40) is attached to the first back side edge (44) and which defines a first side seam length (51), and a second side seam (52) at which the second front side edge (42) is attached to the second back side edge (46) and which defines a second side seam length (53);
an elastomeric front body panel (60) superposed on and attached to the front region (27) of the outer cover layer (24), the front body panel (60) comprising an elastomeric film laminate (62) that extends transversely from the first front side edge (40) to the second front side edge (42), that extends longitudinally along the first front side edge (40) a distance at least 50% of the first side seam length (51), and that extends longitudinally along the second front side edge (42) a distance at least 50% of the second side seam length (53);
an elastomeric back body panel (70) superposed on and attached to the back region (29) of the outer cover layer (24), the back body panel (70) comprising an elastomeric film laminate (72) that extends transversely from the first back side edge (44) to the second back side edge (46), that extends longitudinally along the first back side edge (44) a distance at least 50% of the first side seam length (51), and that extends longitudinally along the second back side edge (46) a distance at least 50% of the second side seam length (53);
an absorbent assembly (58) superposed over the crotch region (38) of the outer cover layer (24); and
wherein the outer cover layer (24) includes a transversely extending front fold (90) that defines the front end edge (26) and a transversely extending back fold (92) that defines the back end edge (28), the garment further comprising a front waist elastic member (93) positioned within the front fold and a back waist elastic member positioned within the back fold (92);
**characterised in that** the front body panel (60) defines a front body panel width (61) that extends transversely from the first front side edge (40) to the second front side edge (42), the front body panel (60) being longitudinally spaced from the front end edge (26) along the entire front body panel width (61); **in that** the back body panel (70) defines a back body panel width (71) that extends transversely from the first back side edge (44) to the second back side edge (46), the back body panel (70) being longitudinally spaced from the back end edge (28) along the entire back body panel width (71);
wherein the elastomeric film laminate (62) in the front body panel (60) and the elastomeric film laminate (72) in the back body panel (70) both comprise an elastomeric film layer (63,73) and a nonwoven layer (64,74), wherein a bodyside surface (65,75) of the elastomeric film layer (63,73) is directly adhered to the nonwoven layer (64,74), wherein a garment-side surface (66,76) of the elastomeric film layer (63,73) is directly adhered to the unitary outer cover layer (24); and wherein a first back leg elastic member (80) and a second back leg elastic member (82) are both at least partially sandwiched between the back body panel (70) and the outer cover layer (24).

2. The garment (20) of claim 1 wherein the front body panel (60) is longitudinally spaced from and distinct from the back body panel (70).

3. The garment (20) of any preceding claim further comprising a first back leg elastic member (80) attached to the outer cover layer (24) adjacent at least a portion of the first leg edge (30), and a second back leg elastic member (82) attached to the outer cover layer (24) adjacent at least a portion of the second leg edge (34).

4. The garment (20) of any preceding claim further comprising a first front leg elastic member (84) attached to the outer cover layer (24) adjacent at least a portion of the first leg edge (30), and a second front leg elastic member (86) attached to the outer cover layer (24) adjacent at least a portion of the second leg edge (34).

5. The garment (20) of any preceding claim wherein at least a portion of the absorbent assembly (58) is superposed over a portion of the front body panel (60).

6. The garment (20) of claim 5 wherein a majority of the area of the portion of the front body panel (60) superposed by the at least a portion of the absorbent assembly (58) comprises a deadened elastomeric film.

7. The garment (20) of any preceding claim wherein at least a portion of the absorbent assembly (58) is superposed over a portion of the back body panel (70).

8. The garment of any preceding claim wherein the first back leg elastic (80) and the second back leg elastic (82) form part of a single, integral back elastic (81) that extends transversely over the absorbent assembly (58).

9. The garment (20) of any preceding claim wherein the front body panel (60) extends longitudinally along the first front side edge (40) a distance at least 80% of the first side seam length (51), and extends longitudinally along the second front side edge (42) a distance at least 80% of the second side seam length (53); and wherein the back body panel (70) extends longitudinally along the first back side edge (44) a distance at least 80% of the first side seam length (51), and extends longitudinally along the second back side edge (46) a distance at least 80% of the second side seam length (53).

10. The garment of any preceding claim wherein at least a portion of the back body panel (70) extends longitudinally inward past both the longitudinally innermost point (54) of the first side seam (51) and the longitudinally innermost point (56) of the second side seam (53).

11. The garment of any preceding claim wherein a least a portion of the front body panel (60) extends longitudinally inward past both the longitudinally innermost point (54) of the first side seam (50) and the longitudinally innermost point (56) of the second side seam (52).

## Patentansprüche

1. Absorptionsfähiges Einwegbekleidungsstück (20), welches eine Längsrichtung (21) und eine Querrichtung (22) definiert, wobei das Bekleidungsstück umfasst:
eine einheitliche äußere Deckschicht (24), wobei die einheitliche äußere Deckschicht (24) definiert:
einen vorderen Endrand (26), einen hinteren Endrand (28) längs gegenüber des vorderen Endrands (26) und einen ersten und zweiten quer gegenüberliegenden Beinrand (30, 34), welche längs zwischen dem vorderen Endrand (26) und dem hinteren Endrand (28) angeordnet sind, wobei jeder Beinrand (30, 34) einen vorderen Endpunkt (31, 35) und einen hinteren Endpunkt (32, 36) aufweist, wobei die äußere Deckschicht (24) einen vorderen Bereich (27) angrenzend an den vorderen Endrand (26), einen hinteren Bereich (29) angrenzend an den hinteren Endrand (28) und einen Schrittbereich (38) bestimmt, der der Länge nach zwischen dem vorderen Bereich (27) und dem hinteren Bereich (29) angeordnet ist;
einen ersten und zweiten quer gegenüberliegenden vorderen Seitenrand (40, 42), wobei der erste vordere Seitenrand (40) sich in der Längsrichtung (21) von dem vorderen Endrand (26) zu dem vorderen Endpunkt (31) des ersten Beinrands (30) erstreckt, und wobei sich der zweite vordere Seitenrand (42) in der Längsrichtung (21) von dem vorderen Endrand (26) zu dem vorderen Endpunkt (35) des zweiten Beinrands (34) erstreckt; und
einen ersten und zweiten quer gegenüberliegenden hinteren Seitenrand (44, 46), wobei der erste hintere Seitenrand (44) sich in der Längsrichtung (21) von dem hinteren Endrand (28) zu dem hinteren Endpunkt (32) des ersten Beinrands (30) erstreckt, und wobei sich der zweite hintere Seitenrand (46) in der Längsrichtung (21) von dem hinteren Endrand (28) zu dem hinteren Endpunkt (36) des zweiten Beinrands (34) erstreckt;
eine erste Seitennaht (50), an welcher der erste vordere Seitenrand (40) an dem ersten hinteren Seitenrand (44) befestigt ist, und welche eine erste Seitennahtlänge (51) definiert, und eine zweite Seitennaht (52), an welcher der zweite vordere Seitenrand (42) an dem zweiten hinteren Seitenrand (46) befestigt ist, und welche eine zweite Seitennahtlänge (53) definiert;
eine elastomere vordere Körperbahn (60), welche mit dem vorderen Bereich (27) der äußeren Deckschicht (24) überlagert und an diesem befestigt ist, wobei die vordere Körperbahn (60) ein elastomeres Filmlaminat (62) umfasst, welches sich quer von dem ersten vorderen Seitenrand (40) zu dem zweiten vorderen Seitenrand (42) erstreckt, welches sich der Länge nach entlang des ersten vorderen Seitenrands (40) über eine Distanz von mindestens 50 % der ersten Seitennahtlänge (51) erstreckt, und welches sich der Länge nach entlang des zweiten vorderen Seitenrands (42) über eine Distanz von mindestens 50 % der zweiten Seitennahtlänge (53) erstreckt;
eine elastomere hintere Körperbahn (70), welche mit dem hinteren Bereich (29) der äußeren Deckschicht (24) überlagert und an diesem befestigt ist, wobei die hintere Körperbahn (70) ein elastomeres Filmlaminat (72) umfasst, welches sich quer von dem ersten hinteren Seitenrand (44) zu dem zweiten hinteren Seitenrand (46) erstreckt, welches sich der Länge nach entlang des ersten hinteren Seitenrands (44) über eine Distanz von mindestens 50 % der ersten Seitennahtlänge (51) erstreckt, und welches sich der Länge nach entlang des zweiten hinteren Seitenrands (46) über eine Distanz von mindestens 50 % der zweiten Seitennahtlänge (53) erstreckt;
eine absorptionsfähige Anordnung (58), welche über dem Schrittbereich (38) der äußeren Deckschicht (24) angeordnet ist; und
wobei die äußere Deckschicht (24) eine sich quer erstreckende vordere Faltung (90) beinhaltet, welche den vorderen Endrand (26) bestimmt und eine sich quer erstreckende hintere Faltung (92) beinhaltet, welche den hinteren Endrand (28) bestimmt, wobei die Bekleidung des Weiteren ein vorderes elastisches Taillenelement (93) umfasst, welches in der vorderen Faltung angeordnet ist, und ein hinteres elastisches Taillenelement, welches in der hinteren Faltung (92) angeordnet ist;
**dadurch gekennzeichnet, dass** die vordere Körperbahn (60) eine vordere Körperbahnbreite (61) bestimmt, welche sich quer von dem ersten vorderen Seitenrand (40) zu dem zweiten vorderen Seitenrand (42) erstreckt, wobei die vordere Körperbahn (60) der Länge nach von dem vorderen Endrand (26) entlang der gesamten vorderen Körperbahnbreite (61) beabstandet ist, dadurch dass die hintere Körperbahn (70) eine hintere Körperbahnbreite (71) bestimmt, welche sich quer von dem ersten hinteren Seitenrand (44) zu dem zweiten hinteren Seitenrand (46) erstreckt, wobei die hintere Körperbahn (70) der Länge nach von dem hinteren Endrand (28) entlang der gesamten hinteren Körperbahnbreite (71) beabstandet ist;
wobei das elastomere Filmlaminat (62) in der vorderen Körperbahn (60) und das elastomere Filmlaminat (72) in der hinteren Körperbahn (70) beide eine elastomere Filmschicht (63, 73) und eine Vliesschicht (64, 74) umfassen, wobei eine körperseitige Fläche (65, 75) der elastomeren Filmschicht (63,73) direkt an der Vliesschicht (64,74) befestigt ist, wobei eine kleidungsseitige Fläche (66, 76) der elastomeren Filmschicht (63,73) direkt an der einheitlichen äußeren Deckschicht (24) befestigt ist; und wobei ein erstes elastisches Element am hinteren Bein (80) und ein zweites elastisches Element am hinteren Bein (82) sich beide zumindest teilweise zwischen einer hinteren Körperbahn (70) und der äußeren Deckschicht (24) befinden.

2. Bekleidung (20) gemäß Anspruch 1, wobei die vordere Körperbahn (60) der Länge nach beabstandet ist von und verschieden ist von der hinteren Körperbahn (70).

3. Bekleidung (20) gemäß einem der vorherigen Ansprüche, welche des Weiteren ein erstes hinteres elastisches Beinelement (80) umfasst, welches an der äußeren Deckschicht (24) angrenzend an mindestens einen Teil des ersten Beinrandes (30) befestigt ist, und ein zweites hinteres elastisches Beinelement (82) umfasst, welches an der äußeren Deckschicht (24) angrenzend an mindestens einen Teil des zweiten Beinrandes (34) befestigt ist.

4. Bekleidung (20) gemäß einem der vorherigen Ansprüche, welche des Weiteren ein erstes vorderes elastisches Beinelement (84) umfasst, welches an der äußeren Deckschicht (24) angrenzend an mindestens einen Teil des ersten Beinrandes (30) befestigt ist, und ein zweites vorderes elastisches Beinelement (86) umfasst, welches an der äußeren Deckschicht (24) angrenzend an mindestens einen Teil des zweiten Beinrandes (34) befestigt ist.

5. Bekleidung (20) gemäß einem der vorherigen Ansprüche, wobei mindestens ein Teil der absorptionsfähigen Anordnung (58) über einem Teil der vorderen Körperbahn (60) überlagert ist.

6. Bekleidung (20) gemäß Anspruch 5, wobei ein Großteil der Fläche des Teils der vorderen Körperbahn (60), welcher durch den mindestens einen Teil der absorptionsfähigen Anordnung (58) überlagert ist, einen stumpfen elastomeren Film umfasst.

7. Bekleidung (20) gemäß einem der vorherigen Ansprüche, wobei mindestens ein Teil der absorptionsfähigen Anordnung (58) über einem Teil der hinteren Körperbahn (70) angeordnet ist.

8. Bekleidung gemäß einem der vorherigen Ansprüche, wobei die erste hintere Beinelastik (80) und die zweite hintere Beinelastik (82) Teile einer einzelnen, integralen hinteren Elastik (81) bilden, welche sich quer über die absorptionsfähige Anordnung (58) erstreckt.

9. Bekleidung (20) gemäß einem der vorherigen Ansprüche, wobei die vordere Körperbahn (60) sich der Länge nach entlang des ersten vorderen Seitenrands (40) über eine Distanz von mindestens 80 % der ersten Seitennahtlänge (51) erstreckt, und sich der Länge nach entlang des zweiten vorderen Seitenrands (42) über eine Distanz von mindestens 80 % der zweiten Seitennahtlänge (53) erstreckt; und wobei die hintere Körperbahn (70) sich der Länge nach entlang des ersten hinteren Seitenrands (44) über eine Distanz von mindestens 80 % der ersten Seitennahtlänge (51) erstreckt, und sich der Länge nach entlang des zweiten hinteren Seitenrands (46) über eine Distanz von mindestens 80 % der zweiten Seitennahtlänge (53) erstreckt.

10. Bekleidung gemäß einem der vorherigen Ansprüche, wobei sich mindestens ein Teil der hinteren Körperbahn (70) der Länge nach nach innen über beide, den längsgelegenen innersten Punkt (54) der ersten Seitennaht (51) und den längsgelegenen innersten Punkt (56) der zweiten Seitennaht (53) erstreckt.

11. Bekleidung gemäß einem der vorherigen Ansprüche, wobei sich mindestens ein Teil der vorderen Körperbahn (60) der Länge nach nach innen über beide, den längsgelegenen innersten Punkt (54) der ersten Seitennaht (50) und den längsgelegenen innersten Punkt (56) der zweiten Seitennaht (52) erstreckt.

## Revendications

1. Vêtement absorbant jetable (20), définissant un sens longitudinal (21) et un sens transversal (22), le vêtement comprenant :
une couche de couverture extérieure unitaire (24), la couche de couverture extérieure unitaire (24) définissant :
un bord d'extrémité avant (26), un bord d'extrémité arrière (28) longitudinalement opposé au bord d'extrémité avant (26) et des premier et second bords de jambe (30, 34) transversalement opposés, positionnés longitudinalement entre le bord d'extrémité avant (26) et le bord d'extrémité arrière (28), chaque bord de jambe (30, 34) ayant un point d'extrémité avant (31, 35) et un point d'extrémité arrière (32, 36), la couverture extérieure (24) définissant une zone avant (27) adjacente au bord d'extrémité avant (26), une zone arrière (29) adjacente au bord d'extrémité arrière (28) et une zone d'entrejambe (38) positionnée longitudinalement entre la zone avant (27) et la zone arrière (29) ;
des premier et second bords latéraux avant (40, 42) transversalement opposés, le premier bord latéral avant (40) s'étendant dans le sens longitudinal (21) depuis le bord d'extrémité avant (26) jusqu'au point d'extrémité avant (31) du premier bord de jambe (30), et le second bord latéral avant (42) s'étendant dans le sens longitudinal (21) depuis le bord d'extrémité avant (26) jusqu'au point d'extrémité avant (35) du second bord de jambe (34) ; et
des premier et second bords latéraux arrière (44, 46) transversalement opposés, le premier bord latéral arrière (44) s'étendant dans le sens longitudinal (21) depuis le bord d'extrémité arrière (28) jusqu'au point d'extrémité arrière (32) du premier bord de jambe (30), et le second bord latéral arrière (46) s'étendant dans le sens longitudinal (21) depuis le bord d'extrémité arrière (28) jusqu'au point d'extrémité arrière (36) du second bord de jambe (34) ;
une première couture latérale (50) au niveau de laquelle le premier bord latéral avant (40) est fixé au premier bord latéral arrière (44) et qui définit une première longueur de couture latérale (51), et une seconde couture latérale (52) au niveau de laquelle le second bord latéral avant (42) est fixé au second bord latéral arrière (46) et qui définit une seconde longueur de couture latérale (53) ;
un pan de corps avant élastomère (60) superposé sur la zone avant (27) de la couche de couverture extérieure (24) et fixé à celle-ci, le pan de corps avant (60) comprenant un stratifié de film élastomère (62) qui s'étend transversalement depuis le premier bord latéral avant (40) jusqu'au second bord latéral avant (42), qui s'étend longitudinalement le long du premier bord latéral avant (40) sur une distance d'au moins 50 % de la première longueur de couture latérale (51), et qui s'étend longitudinalement le long du second bord latéral avant (42) sur une distance d'au moins 50 % de la seconde longueur de couture latérale (53) ;
un pan de corps arrière élastomère (70) superposé sur la zone arrière (29) de la couche de couverture extérieure (24) et fixé à celle-ci, le pan de corps arrière (70) comprenant un stratifié de film élastomère (72) qui s'étend transversalement depuis le premier bord latéral arrière (44) jusqu'au second bord latéral arrière (46), qui s'étend longitudinalement le long du premier bord latéral arrière (44) sur une distance d'au moins 50 % de la première longueur de couture latérale (51) et qui s'étend longitudinalement le long du second bord latéral arrière (46) sur une distance d'au moins 50 % de la seconde longueur de couture latérale (53) ;
un ensemble absorbant (58) superposé sur la zone d'entrejambe (38) de la couche de couverture extérieure (24) ; et
dans lequel la couche de couverture extérieure (24) comprend une pliure avant à étendue transversale (90), qui définit le bord d'extrémité avant (26) et une pliure arrière à étendue transversale (92), qui définit le bord d'extrémité arrière (28), le vêtement comprenant en outre un élément élastique de taille avant (93), positionné dans la pliure avant et un élément élastique de taille arrière, positionné dans la pliure arrière (92) ;
**caractérisé en ce que** le pan de corps avant (60) définit une largeur de pan de corps avant (61) qui s'étend transversalement depuis le premier bord latéral avant (40) jusqu'au second bord latéral avant (42), le pan de corps avant (60) étant longitudinalement espacé du bord d'extrémité avant (26), sur toute la largeur du pan de corps avant (61) ; **en ce que** le pan de corps arrière (70) définit une largeur de pan de corps arrière (71) qui s'étend transversalement depuis le premier bord latéral arrière (44) jusqu'au second bord latéral arrière (46), le pan de corps arrière (70) étant longitudinalement espacé du bord d'extrémité arrière (28), sur toute la largeur du pan de corps arrière (71) ;
dans lequel le stratifié de film élastomère (62) dans le pan de corps avant (60) et le stratifié de film élastomère (72) dans le pan de corps arrière (70) comprennent tous deux une couche de film élastomère (63, 73) et une couche non tissée (64, 74), une surface côté corps (65, 75) de la couche de film élastomère (63, 73) étant directement collée sur la couche non tissée (64, 74), une surface côté vêtement (66, 76) de la couche de film élastomère (63, 73) étant directement collée sur la couche de couverture extérieure unitaire (24) ; et dans lequel un premier élément élastique de jambe arrière (80) et un second élément élastique de jambe arrière (82) sont tous deux au moins partiellement pris en sandwich entre le pan de corps arrière (70) et la couche de couverture extérieure (24).

2. Vêtement (20) selon la revendication 1, dans lequel le pan de corps avant (60) est longitudinalement espacé et distinct du pan de corps arrière (70).

3. Vêtement (20) selon l'une quelconque des revendications précédentes, comprenant en outre un premier élément élastique de jambe arrière (80), fixé à la couche de couverture extérieure (24), de manière adjacente à au moins une partie du premier bord de jambe (30), et un second élément élastique de jambe arrière (82), fixé à la couche de couverture extérieure (24), de manière adjacente à au moins une partie du second bord de jambe (34).

4. Vêtement (20) selon l'une quelconque des revendications précédentes, comprenant en outre un premier élément élastique de jambe avant (84), fixé à la couche de couverture extérieure (24), de manière adjacente à au moins une partie du premier bord de jambe (30), et un second élément élastique de jambe avant (86), fixé à la couche de couverture extérieure (24), de manière adjacente à au moins une partie du second bord de jambe (34).

5. Vêtement (20) selon l'une quelconque des revendications précédentes, dans lequel au moins une partie de l'ensemble absorbant (58) est superposée sur une partie du pan de corps avant (60).

6. Vêtement (20) selon la revendication 5, dans lequel une majorité de la zone de la partie du pan de corps avant (60) recouverte par ladite au moins une partie de l'ensemble absorbant (58) comprend un film élastomère amorti.

7. Vêtement (20) selon l'une quelconque des revendications précédentes, dans lequel au moins une partie de l'ensemble absorbant (58) est superposée sur une partie du pan de corps arrière (70).

8. Vêtement selon l'une quelconque des revendications précédentes, dans lequel le premier élastique de jambe arrière (80) et le second élastique de jambe arrière (82) forment un élastique arrière unique formé d'un seul tenant (81) qui s'étend transversalement sur l'ensemble absorbant (58) .

9. Vêtement (20) selon l'une quelconque des revendications précédentes, dans lequel le pan de corps avant (60) s'étend longitudinalement le long du premier bord latéral avant (40), sur une distance d'au moins 80 % de la première longueur de couture latérale (51) et s'étend longitudinalement le long du second bord latéral avant (42) sur une distance d'au moins 80 % de la seconde longueur de couture latérale (53) ; et dans lequel le pan de corps arrière (70) s'étend longitudinalement le long du premier bord latéral arrière (44) sur une distance d'au moins 80 % de la première longueur de couture latérale (51) et s'étend longitudinalement le long du second bord latéral arrière (46) sur une distance d'au moins 80 % de la seconde longueur de couture latérale (53).

10. Vêtement selon l'une quelconque des revendications précédentes, dans lequel au moins une partie du pan de corps arrière (70) s'étend longitudinalement vers l'intérieur, au-delà du point positionné longitudinalement le plus à l'intérieur (54) de la première couture latérale (51) et du point positionné longitudinalement le plus à l'intérieur (56) de la seconde couture latérale (53).

11. Vêtement selon l'une quelconque des revendications précédentes, dans lequel au moins une partie du pan de corps avant (60) s'étend longitudinalement vers l'intérieur, au-delà du point longitudinalement le plus à l'intérieur (54) de la première couture latérale (50) et du point longitudinalement le plus à l'intérieur (56) de la seconde couture latérale (52).
